# EUROPEAN PATENT SPECIFICATION

(11) **EP 1 298 348 B1**
(45) Date of publication and mention of the grant of the patent: **07.09.2005**
(21) Application number: 02021739.4
(22) Date of filing: 25.09.2002
(51) Int. Cl.: F16F 1/38, B62D 21/11

(54) **Bush, group of bush, suspension cross-member, vehicle, method for mounting a bush and method for mounting a group of bushes**
Buchse, Einheit von Buchse, Querträger einer Aufhängung und Fahrzeug, Methode zur Montage einer Buchse und Methode zur Montage einer Gruppe von Buchsen
Manchon, ensemble de manchons, traverse de suspension et véhicule, méthode de montage d'un manchon et méthode de montage d'un groupe de manchons

(30) Priority: 28.09.2001 JP 2001299300
(43) Date of publication of application: 02.04.2003
(73) Proprietor: Mazda Motor Corporation, Hiroshima 730-8670 (JP)
(72) Inventor: Yamamoto, Tadanobu, Aki-gun, Hiroshima 730-8670 (JP); Sano, Susumu, Aki-gun, Hiroshima 730-8670 (JP); Yoshimura, Tadashi, Aki-gun, Hiroshima 730-8670 (JP)
(74) Representative: Müller-Boré & Partner Patentanwälte

(56) References cited:
- US-A- 4 109 979
- PATENT ABSTRACTS OF JAPAN vol. 2000, no. 08, 6 October 2000 (2000-10-06) & JP 2000 145854 A (TOYOTA MOTOR CORP), 26 May 2000 (2000-05-26)
- PATENT ABSTRACTS OF JAPAN vol. 2000, no. 20, 10 July 2001 (2001-07-10) & JP 2001 071944 A (TOYOTA MOTOR CORP), 21 March 2001 (2001-03-21)

## Description

The present invention relates to a bush, more particularly, to a bush and a group of bush for mounting a suspension cross-member on a vehicle, to a suspension cross-member fitted with such bushes, to a vehicle on which such a suspension cross-member is mounted, to a method for mountiong such a bush and to a method for mounting such a group of bushes.

Conventionally, bushes are used to mount a suspension cross-member to the bottom of a vehicle body. The structure of such bushes is disclosed in Japanese Patent Publication No. 2001-71944 for example, which comprises an outer member, an inner member which is inserted into the outer member and through which a stud on the body projects downwardly, and a elastic member disposed between the outer member and the inner member. In the structure, the bushes have an axial length sufficient for penetrating the cross-member and are press-fitted into respective mounting holes from one direction. To mount the cross-member to the body, the cross-member is fitted with the bushes, positioned in place with respect to the body so that the body studs are inserted into the inner member, and then secured to the body via nuts fitted onto the studs. The previous adjustment of a spring constant of the elastic member of the bush achieves intended ride comfort and handling stability of the vehicle.

In a meantime, the application of preload to the elastic member is well known for satisfying the needs of the ride comfort and the handling stability. For example, in the bush structure constituted as described above, the elastic member has an extended axial length so as to protrude in the axial direction or in the vertical direction by a predetermined amount, and the nut is screwed until the elastic member is compressed between the body or the cross-member and the nut. Thus, after the cross-member is mounted on the body, the elastic member is provided with the predetermined amount of preload. According to the structure as above, the elastic member is interposed between the body and the cross-member so as to be compressed in the vertical direction, so that the frequency dependence is reduced to improve the ride comfort, and the intended spring constant is produced in the elastic member. That is, the proper axial preload of the elastic member beneficially provides the vehicle with the increased ride comfort and handling stability at once.

The cross-member is supported on the body via a plurality of bushes, each of which is press-fitted into the cross-member and secured to the body. Accordingly, the variation in the amount of preload among the respective mounting portions or bushes may cause the degradation in the handling stability and ride comfort. To avoid the degradation, the accurate amount of preload should be applied to each bush in the axial direction, thus measures are required for adjusting the screwing amount of each nut and for ensuring that the elastic member adequately deforms at the abutting portion on the body and the cross-member. Even with the measures described above, however, the variation in dimensional accuracy inevitably occurs in the cross-member or the bushes, which adversely affects the accuracy of the amount of the preload. Though the problem can be eliminated by precise management of the dimensional accuracy of parts, it causes the parts cost to rise and the production efficiency to decrease.

In addition, as disclosed in Japanese Patent Publication 2000-145854 for example, the bushes are typically fitted to attaching holes in suspension parts by press-fit operation. In the press-fit operation, a jig is used, which displaces the bush in the fitting direction while abutting on the bush. However, the jig may deform the bush in the presence of friction between the bush and the attaching hole, which also results in the variation in the amount of preload.

In view of the problem above, an object of the present invention is to achieve the excellent handling stability and ride comfort of the vehicle at once with the reduced cost.

This object is solved according to the invention by a bush according to claim 1, by a group of bushes according to claim 6, by a suspension cross-member according to claim 5 or claim 7, by a vehicle according to claim 8, by a method for mounting a bush according to claim 9 and by a method for mounting a group of bushes according to claim 10. Preferred embodiments of the present invention are subject of the dependent claims.

Thus, according to the present invention, the excellent handling stability and ride comfort is achieved in a vehicle at once, by the provision of a bush for mounting the cross-member to the bottom of the body which decreases the amount of the variation in preload for easily and reliably attaining the intended amount of preload with the reduced cost.

According to the present invention, there is provided a bush which can be press-fitted into a top aperture and a bottom aperture of a bush-attaching hole penetrating a suspension cross-member and fixed or fixable to a vehicular body by a stud and a nut for mounting the suspension cross-member to the bottom of the vehicular body, comprising, an outer member including an outer main body in substantially cylindrical shape with an axial length shorter than a half of the axial length of the attaching hole of the suspension cross-member and an outer flange extending in the radially outward direction from an axial end (or end portion) of the outer main body, an inner member including an inner main body in substantially cylindrical shape with an axial length longer than a half of the axial length of the attaching hole of the suspension cross-member and with an external diameter smaller than the internal diameter of the outer main body, an inner flange extending in the radially outward direction from an axial end (or end portion) of the inner main body with an external diameter larger than the internal diameter of the outer main body, and a through hole through which the stud can be inserted, and the inner member being coaxially inserted or insertable into the outer member so that the inner flange and the outer flange are on the same side with respect to the axial direction, an elastic member disposed or arrangeable between the inner member and the outer member and adhered to the inner main body, inner flange, the outer main body, and the outer flange, wherein the outer member, the inner member and/or the elastic member are formed such that, when the bushes are press-fitted into the top aperture and the bottom aperture of the bush-attaching hole until the outer flanges contact with the suspension cross-member before the bushes are fixed by the stud and the nut, the elastic members are substantially free from elastic deformation and provide a predetermined clearance between the other axial ends of the inner members, and when the bushes are fixed to the body by the stud and the nut, the elastic members are elastically deformed by tightening the nut fitted onto the stud so that the inner members are axially displaced with respect to the outer members to abut on each other.

Accordingly, for mounting the cross-member with the fastening means, the bushes are or can be press-fitted into the top aperture and the bottom aperture of the bush-attaching hole, and the fastening means is tightened until the ends of the inner members of the bushes press-fitted abut on each other. Therefore, the operation as above can easily provide an intended axial preload corresponding to the predetermined clearance in the bushes in a reliable manner with little or no variation in the amount of the preload among the bushes in respective attaching holes, after the cross-member is fixed to the body.

Preferably, the outer member further may include a jig-abutting portion provided in the outer flange, on which a jig can abut for press-fitting the bush into an attaching hole in the suspension cross-member, and a reinforcing portion formed as a bending portion of the outer flange, for preventing deformation of the outer member when the bush is press-fitted with the jig.

Accordingly, in the press-fit operation, the reinforcing portion can prevent deformation of the outer member due to the friction between the bush and the bush-attaching hole, which ensures the accurate amount of preload provided in the bush.

Further preferably, the jig-abutting portion may be a substantially planar surface which continues to and is at substantially right angle with the outer main body in a cross section including the central axis of the bush.

Accordingly, in the press-fit operation, the jig can press the portion close to the outer main body of the outer flange, so that the deformation of the outer flange is avoided in combination with the effect of the reinforcing portion.

Still further preferably, the reinforcing portion may include a slanted surface which is at an angle larger than the right angle or at an abtuse angle with the outer main body, and the inner flange may include a tilt which confronts or substantially faces the slanted surface substantially in parallel.

Accordingly, the elastic member is or can be compressed by two surfaces confronting in parallel with each other, so that the accuracy in preload provided in the bush increases. Moreover, such an advantage is available by use of a part of the reinforcing portion.

According to the present invention, there is further provided a suspension cross-member, including, a bush-attaching hole which penetrates the suspension cross-member to form a top aperture and a bottom aperture on the top surface and the bottom surface of the suspension cross-member, respectively, and a pair of bush according to the present invention or an embodiment thereof, which are press-fitted into the top aperture and the bottom aperture, respectively.

According to the present invention, there is still further provided a group of bushes according to the present invention or an embodiment thereof, each of the bushes can be press-fitted into top apertures and bottom apertures of at least three bush-attaching holes penetrating the suspension cross-member and fixed to a vehicular body by at least three pairs of a stud and a nut for mounting the suspension cross-member to the body, the number of the bushes consisting the group being equal to twice the number of the bush-attaching holes, wherein, the group of the bushes is composed of a first bush, a second bush, and third bushes, the first bush includes a through hole with an internal diameter approximately equal to an external diameter of the stud, the second bush is substantially identical to the first bush excluding that the through hole of the second bush is oblong in cross-section which is expand in one radial direction from the through hole of the first bush, and the third bushes are substantially identical to the first bush excluding that the through hole of the third bushes are circular which is expanded in all radial directions from (or has a larger inner diameter than) the through hole of the first bush.

Accordingly, in addition to the advantage that an intended axial preload corresponding to the predetermined clearance in the bushes is provided in a reliable manner with little or no variation in the amount of the preload among the bushes in respective attaching holes after the cross-member is fixed to the body, the another advantage is attained in the following manner:
1. In the mounting operation of the suspension cross-member after the bushes are press-fitted, the first bush positions a point of the cross-member in place at the first bush-attaching hole,
2. At the second bush-attaching hole, the through hole of the second bush allows the misalignment in the first direction along the line connecting the first attaching hole with the second attaching hole and positions the cross-member in place in the second direction perpendicular to the first direction. That is, the bushes in the first bush-attaching hole and the bushes in the second bush-attaching hole position the cross-member in place with respect to the body.
3. Moreover, at the third bush-attaching holes, the through holes of the third bushes allow the misalignment in both the first and second directions. Accordingly, the assignment of the group of the bushes as above prevents the horizontal stress of fixed bushes.

According to the present invention, there is provided a suspension cross-member, including, at least three bush-attaching holes which penetrate the suspension cross-member to form top apertures and bottom apertures on the top surface and the bottom surface of the suspension cross-member, respectively, wherein, each bush of the group according to the present invention or an embodiment thereof is assigned and press-fitted in such a manner that: the first bush is press-fitted into one of the top aperture and the bottom aperture of the first bush-attaching hole of the at least three bush-attaching holes, the second bush is press-fitted into one of the top aperture and the bottom aperture of the second bush-attaching hole of the at least three bush-attaching holes so that the expanded direction of the through hole is oriented substantially towards the location of the first bush-attaching hole, the third bushes are press-fitted into the other aperture of the first bush-attaching hole, the other aperture of the second bush-attaching hole, and both the top apertures and the bottom apertures of the other bush-attaching holes.

According to the present invention, there is further provided a vehicle on which the suspension cross-member according to the present invention or an embodiment thereof is mounted, wherein, the suspension cross-member is fixed to the vehicular body via the stud and the nut and the bushes press-fitted into the bush-attaching holes so that the other axial ends of the inner members of the bushes in the respective top apertures and the other axial ends the inner members of the respective bushes in the bottom apertures abut on each other.

According to the invention, there is still further provided a method for mounting or assembling a bush according to the invention or an embodiment thereof into a top aperture and a bottom aperture of a bush-attaching hole penetrating a suspension cross-member and fixable to a vehicular body by a stud and a nut for mounting the suspension cross-member to the bottom of the vehicular body, comprising the following steps:
press-fitting the bushes into the top aperture and the bottom aperture of the bush-attaching hole until the outer flanges contact with the suspension cross-member before the bushes are fixed by the stud and the nut, whereby the elastic members are substantially free from elastic deformation and provide a predetermined clearance between the other axial ends of the inner members, and
elastically deforming the elastic members by tightening the nut fitted onto the stud so that the inner members are axially displaced with respect to the outer members to abut on each other for fixing the bushes to the body by the stud (42) and the nut.

According to the invention, there is still further provided a method for mounting a group of bushes according to the invention or an embodiment thereof to a suspension cross-member having at least three bush-attaching holes which penetrate the suspension cross-member to form top apertures and bottom apertures on the top surface and the bottom surface of the suspension cross-member, respectively, comprising the following steps:
press-fitting the first bush into one of the top aperture and the bottom aperture of the first bush-attaching hole of the at least three bush-attaching holes,
press-fitting the second bush into one of the top aperture and the bottom aperture of the second bush-attaching hole of the at least three bush-attaching holes so that the expanded direction of the through hole is oriented substantially towards the location of the first bush-attaching hole, and
press-fitting the third bushes into the other aperture of the first bush-attaching hole, the other aperture of the second bush-attaching hole, and both the top apertures and the bottom apertures of the other bush-attaching holes.

These and other objects, features and advantages of the present invention will become more apparent upon reading of the following detailed description of preferred embodiments and accompanying drawings.
FIG. 1 is a top plan view of a front suspension cross-member mounted to a body via bushes according to the present invention.
FIG. 2 is a plan view of bushes according to the present invention.
FIG. 3 is a cross-sectional view taken along A - A in FIG. 2.
FIG. 4 is a cross-sectional view taken along B - B in FIG. 2.
FIG. 5 is a cross-sectional view of bushes in press-fitting operation, corresponding to a cross-sectional view taken along B - B in FIG. 2.
FIG. 6 is a cross-sectional view of press-fitted bushes, corresponding to a cross-sectional view taken along A - A in FIG. 2.
FIG. 7 is a cross-sectional view of bushed secured to the body, corresponding to a cross-sectional view taken along A - A in FIG. 2.

A preferred embodiment of the present invention will now be described with reference to the drawings.

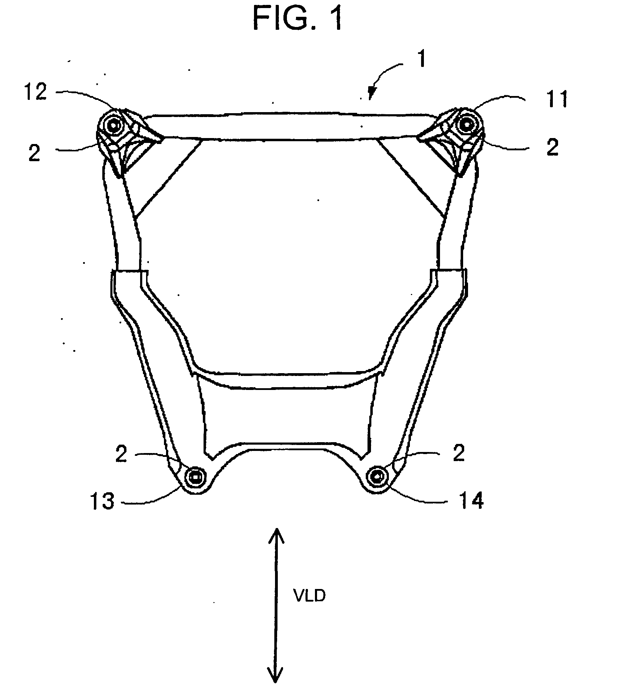

FIG. 1 is a top plan view of a front suspension cross-member (referred to as cross-member) 1 whish is mounted to the body via three kinds of bushes or sleeves 2a, 2b, and 2c (each of which is generally indicated as 2) according to a preferred embodiment of the present invention. As shown, the cross-member 1 is substantially rectangular in shape comprising flames with a closed cross-section. The cross-member 1 is formed with four bush-attaching holes 11 to 14 into which bushes 2 are press-fitted. Though FIG. 1 shows bushes in the top surface of the cross-member 1, bushes 2 are also press-fitted into the bottom surface of the cross-member 1. In the meantime, a vehicular body (not shown) is provided with four studs projecting therefrom, which are correspondingly arranged with respect to the location of the bush 2. Thus, the cross-member 1, fitted with the bushes 2, is fixed to the body via the bushes by securing the bushes 2 onto the body with the studs and nuts.

The bushes 2 to be press-fitted into the bush-attaching holes 11 to 14 are of split-type, which comprises a pair of halves split in the middle with respect to the axis before press-fitted. In other words, a pair of bushes is each press-fitted into the top aperture and the bottom aperture of the respective bush-attaching holes 11 to 14 of the cross-member 1.

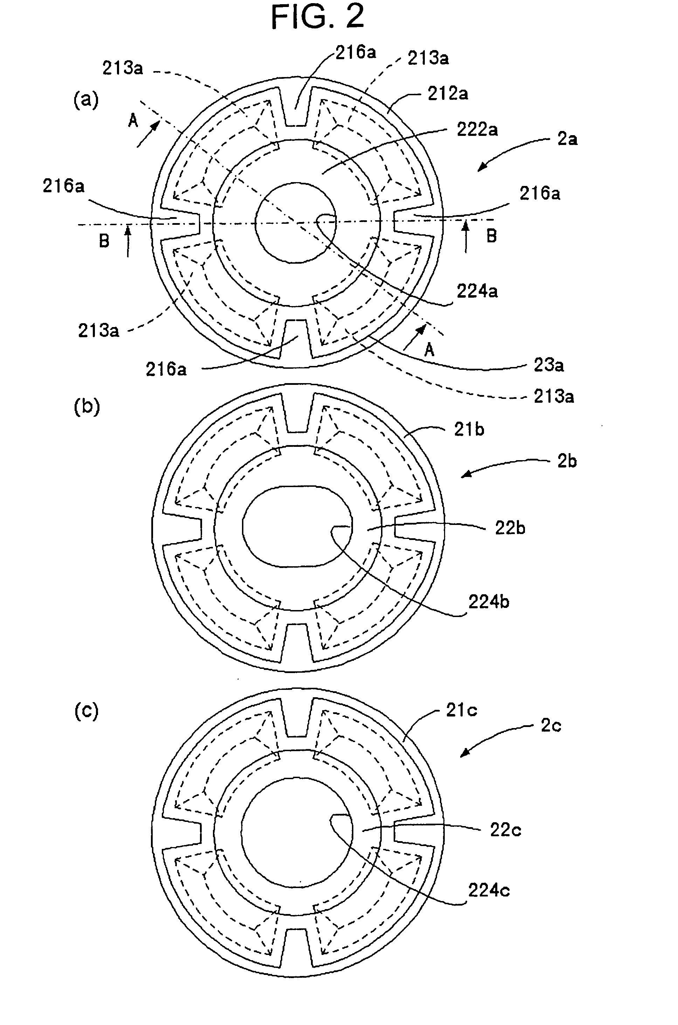

The constitution of the bush 2 will now be described with reference to FIG. 2 to FIG. 4.

As shown in FIGS. 2 (a) to (c) illustrating plan views of the bush 2, three kinds of bushes 2a to 2c are prepared which have through holes 224a, 224b, and 224c different from one another in shape, respectively. The difference will be described in detail below. As an example of the bushes 2, the constitution of the bush 2a will now be described hereinbelow with reference to FIG. 2 (a), because the constitution is common to the three except for the through holes 224a to 224c.

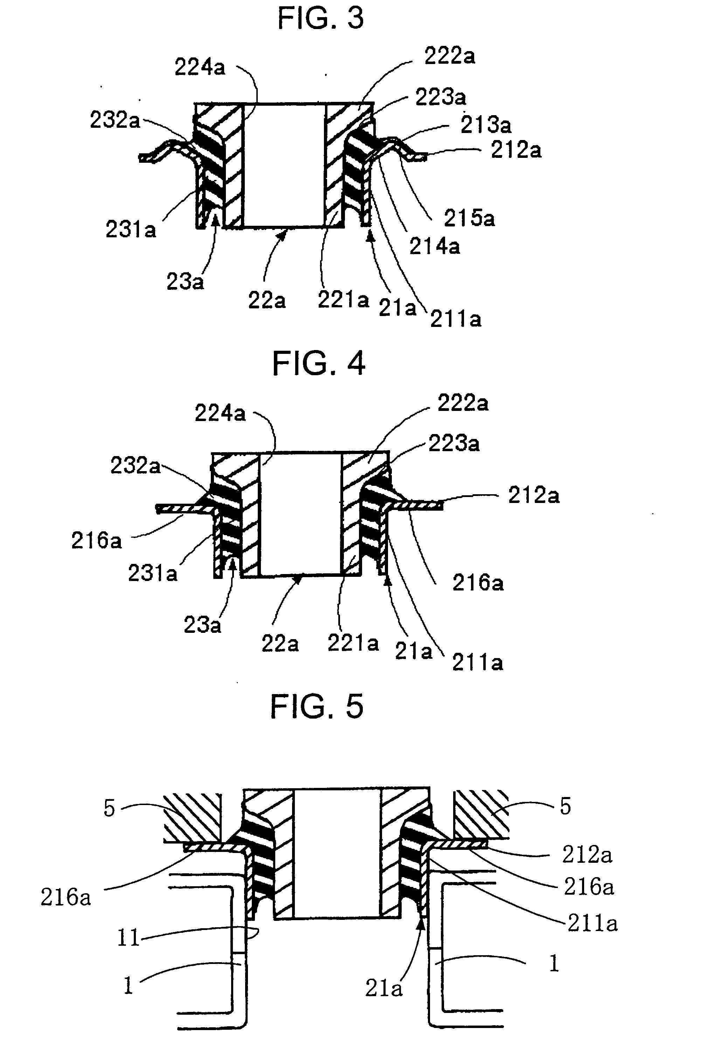

As shown in FIG. 3 illustrating a cross-sectional view taken along A - A in FIG. 2, and in FIG. 4 illustrating a cross-sectional view taken along B - B in FIG. 2, the bush 2a comprises a metallic outer member 21a, a metallic inner member 22a, and an elastic or resilient member 23a. The outer member 21a includes a cylindrical portion. The inner member 22a includes a cylindrical portion with an external diameter smaller than an internal diameter of the outer member 21a and with the through hole 224a through which the stud for mounting the cross-member is inserted. The elastic member is disposed between and adhered to both the outer member 21a and inner member 22a.

As shown in FIG. 3 and FIG. 4, the outer member 21a includes an outer main body 211a in a cylindrical shape with an internal diameter larger than the external diameter of the inner member 22a, and an outer flange 212a extending in the radially outward direction from an axial end of the main body 211a. The axial length of the outer member 21a is smaller than a half of the thickness of a portion where the bush-attaching holes 11 to 14 for the bush 2a are formed in the cross-member 1. The outer flange 212a is partially formed with reinforcing portions 213a substantially along the circumferential direction of the flange. Particularly, as shown in FIG. 2 in which the edge lines of the reinforcing portions 213a are indicated by broken lines, a plurality of, preferably four reinforcing portions 213a are provided substantially equally spaced apart along the circumferential direction of the outer flange 212a. Moreover, as shown in FIG. 3, the reinforcing portions 213a comprises an inner tilt 214a and outer tilt 215a which constitute bent portions of the flange 212a, which protrudes away from the outer main body 211a. Further, as shown in FIG. 4, a substantially flat surface between the four reinforcing portions 213a formed substantially equally spaced apart along the circumferential direction is approximately perpendicular to the outer main body 211a to provide a jig-abutting portion 216a.

As shown in FIG. 3 and FIG. 4, the inner member 22a includes a through hole 224a through which the stud for mounting the cross-member 1 is inserted, an inner main body 221a in a cylindrical shape, and an inner flange 222a having an external diameter larger than the internal diameter of the outer main body 211a and extending in the radially outward direction from an axial end or end portion of the inner main body 221a. The inner member 22a is substantially coaxially inserted into the outer member 21a so that the inner flange 222a and the outer flange 212a are on the same side with respect to the axial direction. On a surface on the side of inner main body 221a of the inner flange 222a, a slanted surface 223a is provided along the circumferential direction, which continues to the inner main body 221a and is at the predetermined angle different from 0° or 180°, preferably an obtuse angle with respect to the inner main body 221a. The slanted surface 223a is approximately parallel with the inner tilt 214a of the outer flange 212a. The axial length of the inner member 22a is longer than a half of the thickness of the portion where the bush-attaching holes 11-14 for this bush 2a in the cross-member 1.

Between the inner member 22a and the outer member 21a, the elastic member 23a, made from a resilient material, e.g. rubber, is at least partly disposed. As shown in FIG. 3 and FIG. 4, the elastic member includes an elastic main body 231a and an extending portion 232a which extends from an axial end or end portion of the elastic member 23a. The elastic member 23a is adhered to the inner member 22a and the outer member 21a preferably by bond applied over the contact surface thereof. The radially inner surface of the extending portion 232a substantially contacts with the outer surface of the inner main body 221a and the slanted surface 223a, and the radially outer surface of the extending portion 232a substantially contacts with the inner surface of the outer main body 211a and the reinforcing portion 213a of the outer flange 212a. Additionally, as shown in FIG. 2, the extending portion 232a is provided with covering portions which cover the reinforcing portion 213a. The covering portions of the elastic member 23a are adhered to the reinforcing portions 213a, which increases the peel resistance of the elastic member 23a from the outer member 21a. The jig-abutting portion 216a of the outer flange 212a, or the portions free of the reinforcing portion 213a in the outer flange 212a, are not covered with the elastic member.

This embodiment uses three kinds of bushes with the common structure as above and with the through holes of the inner member different in shape for mounting the cross-member 1 to the body. The shapes of through holes 224a, 224b, and 224c, and the assignment of the bushes 2a, 2b, and 2c to the cross-member 1 will now be described with reference to FIG. 2 and FIG. 1.

As shown in FIG. 2 (a), the through hole 224a is formed in the inner member 22a of the first bush 2a. The cross-section of the through hole 224a is substantially circular and substantially equal to the stud for mounting the cross member 1 on the body in diameter. As shown in FIG. 2 (b), the through hole 224b is formed in the inner member 22b of the second bush 2b. The cross section of the through hole 224b is oblong or elongated, which is expanded or elongated in a direction perpendicular to the axial direction of the through hole from the through hole 224a of the first bush 2a. As shown in FIG. 2 (c), the through hole 224c is formed in the inner member 22c of the third bush 2c. The cross-section of the through hole 224c is substantially circular, which is expanded in all radial directions from the internal diameter of the through hole 224a of the first bush 2a. In other words, the internal diameter of the through hole 224c is larger than that of the through hole 224a. The outer member 21a, 21b, and 21c, and the elastic member 23a, 23b, and 23c are identical among the three bushes.

The three kinds of the bushes 2a, 2b, and 2c are assigned to the bush-attaching holes 11 to 14 shown in FIG. 1 in the predetermined manner as follows:
1. In a reference bush-attaching hole 11 which can be arbitrarily selected from the four holes 11-14, the first bush 2a is press-fitted into one of the top aperture and the bottom aperture, and the third bush 2c is press-fitted into the other.
2. In the bush-attaching hole 12 which is laterally proximate to the reference attaching hole 11 with respect to a vehicular longitudinal direction VLD, the third bush 2c is press-fitted into one of the top aperture and the bottom aperture, and the second bush 2b is press-fitted into the other so that the expanded direction of the through holes of the bush 2b is oriented towards the location of the first bush 2a, or oriented in the vehicular lateral direction (or in a direction substantially normal to the vehicular longitudinal direction VLD).
3. In the other bush-attaching holes 13 and 14, the third bushes 2c are press-fitted into both the top apertures and the bottom apertures.
That is, for mounting the cross-member 1, a group of eight bushes 2a-2c is used, which consists of one first bush 2a, one second bush 2b, and six third bushes 2c.

With the assignment of the first bush 2a, the second bush 2b, and the third bush 2c described as above, the cross-member 1 is positioned in the following manner: At the reference bush-attaching hole 11, the first bush 2a positions a point of the cross-member 1 in place. At the bush-attaching hole 12 laterally proximate to the reference hole, the through hole 224b of the second bush 2b allows the lateral misalignment between the body stud and the second bush 2b because the expanded direction of the through hole 224b is oriented in the vehicular lateral direction, and positions the cross-member 1 in place in the vehicular longitudinal direction (or in a direction substantially normal to the vehicular longitudinal direction VLD). Thus, cross-member 1 is positioned in place with respect to the body. At the other bush-attaching holes 13, 14, the through holes 224c of third bushes 2c allow both the lateral and longitudinal misalignments between the body stud and the third bushes 2c because the internal diameter of the third bush 2c is expanded in all radial directions. Accordingly, the bushes 2a-2c press-fitted into the holes 11 to 14 of cross-member 1 are properly positioned with respect to the studs on the body frame, and the misalignments between the bushes and the studs are allowed at all the bush-attaching holes 11-14.

As a result, the cross-member 1 is positioned in place with respect to the body and the interference of the studs with the bushes 2 is avoided, so that the bushes 2 are prevented from being horizontally biased after the cross-member 1 is mounted to the body. In addition, only three kinds of bushes 2a-2c are required for positioning the cross-member 1 in place.

The press-fitting operation of the bushes 2 into the cross-member 1 and the mounting operation of the cross-member 1 onto the body will now be described with reference to the FIG. 5 to FIG. 7 showing the bush-attaching hole 11, by way of example.

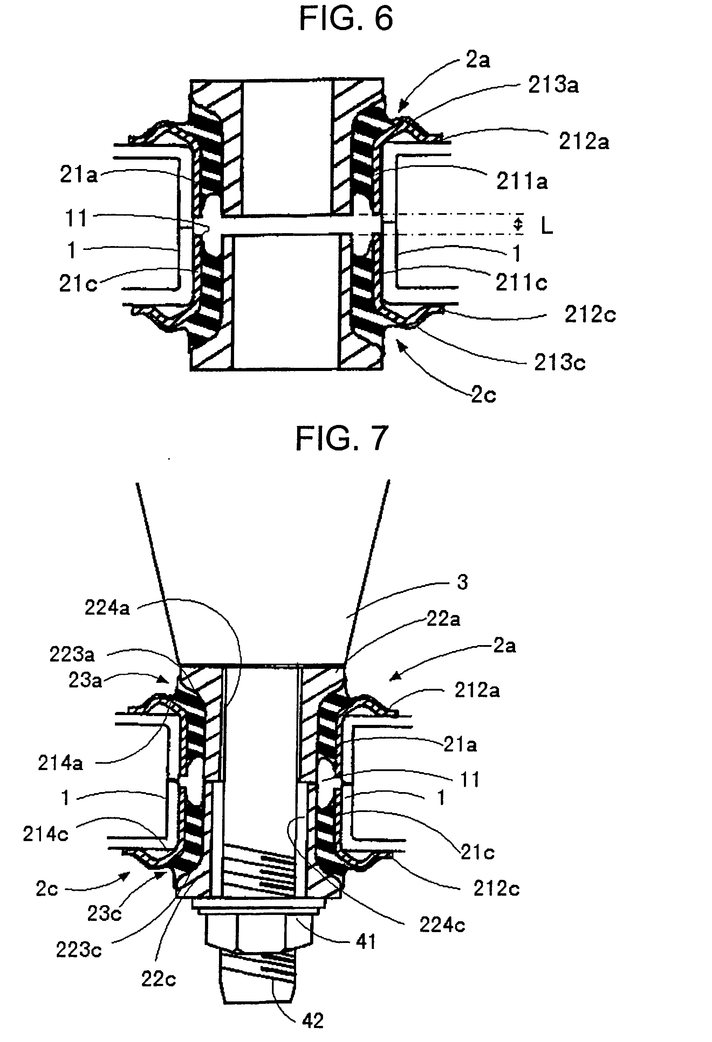

The bushes 2a, 2b, and 2c are assigned in the manner as above and press-fitted into the bush-attaching holes 11 to 14 of the cross-member 1, respectively. With regard to the attaching hole 11 for example, as shown in FIG. 6 showing a cross-sectional view taken along A - A in FIG. 2, the bush 2a is press-fitted into the top aperture and the bush 2c is press-fitted into the bottom aperture. Then, the cross-member 1 fitted with the bushes 2a to 2c is positioned so that the studs 42 on the body 3 are inserted into the through holes 224a to 224c of the bush 2a to 2c. Thereafter, the nuts 41 are fitted onto the studs 42 for fixing the bushes 2a, 2c, as a result, the cross-member 1 is fixed to the body 3 via the bushes 2a, 2c. With regard to the attaching hole 11 for example, the condition after the cross-member 1 is fixed to the body is illustrated in FIG. 7 showing a cross-sectional view taken along A - A in FIG. 2. Before fixed to the body 3, the cross-member 1 is previously fitted with suspension arms supporting wheel hubs, coil dampers, stabilizers, or other parts (not shown).

As shown in FIG. 5, in the press-fitting operation of the bush 2a into the bush-attaching hole 11, the jig 5 abuts onto the jig-abutting portion 216a in the outer flange 212a, and displaces the bush 2a until the outer flange 212a substantially comes into contact with the top surface of the cross-member 1. Likewise, as for the bush 2c, the jig 5 abuts onto the jig-abutting portion 216c in the outer flange 212c, and displaces the bush 2c until the outer flange 212c comes into contact with the bottom surface of the cross-member 1.

Accordingly, with regard to the bush 2a in the top aperture for example, the jig 5 presses the portion close to the central axis of the bush 2a between the reinforcing portions 213a of the outer flange 212a. Thus, the deformation of the outer member 21a, particularly, the deformation at the connecting portion of the outer main body 211a and the outer flange 212a due to the friction and/or pressing during the press-fitting operation is prevented in conjunction with the effect of the reinforcing portion 213a. Likewise, with regard to the bush 2c in the bottom aperture, the deformation at the connecting portion of the outer main body 211c and the outer flange 212c is prevented. Then, after the cross-member 1 is fitted with the bushes 2a and 2c before mounted to the body, there exists a clearance L between both the inner members, as shown in FIG. 6.

With regard to the other bush-attaching holes 12 to 14, as with the bush-attaching hole 11, the bushes 2b and 2c are assigned as above and press-fitted, then there exist the predetermined clearances between the inner members 22b, 22c after the bushes 2b, 2c are press-fitted.

The cross-member 1 fitted with the bushes 2a to 2c and the parts as above are fixed to the body 3 via the bushes 2a to 2c. In the bush-attaching hole 11 for example, as shown in FIG. 7, the stud 42 is inserted through the respective through holes 224a and 224c of the press-fitted bushes 2a and 2c, and then the nuts 41 is fitted onto the stud 42 for fixing the bushes 2 so that the cross-member 1 is fixed to the body 3 via the bushes 2a and 2c. The nut 41 is screwed so as to cause the inner member 22a of the upper bush 2a and the inner member 22c of the lower bush 2c to be displaced with respect to the outer member 21a and 21c, until the inner members abut on each other. After the nut 41 is screwed as above, the elastic member 23a of the upper bush 2a and the elastic member 23c and the lower bush 2c are compressed vertically between the inner member 22a and the outer member 21a, and between the inner member 22c and the outer member 21c by the predetermined amount corresponding to the clearance L, respectively. Thus, there is provided axial or vertical preload corresponding to the compression in the bushes 2a, 2c.

Accordingly, whenever the distance between the top surface and the bottom surface of the portion of the cross-member 1 with which the outer flange 212a and 212c contact is accurate, the predetermined vertical preload corresponding to the compression by the clearance L is easily and reliably applied to the bush 2a and 2c, by screwing the nut 41 until the inner member 22a of the bush 2a and the inner member 22c of the bush 2c abut on each other.

With regard to the other bush-attaching holes 12 to 14, as with the bush-attaching hole 11, the studs are inserted through the through hole 224b of the bush 2b and the through hole 224c of and the bush 2c, and the nut is screwed until the inner members abut on each other.

Accordingly, the setting the clearance L to the amount for the proper preload enables the bushes 2b, 2c press-fitted into the respective bush-attaching holes 12-14 to be provided with the proper amount of preload with little or no variation among the bushes 2b, 2c. Additionally, the slanted surface 223c of the inner flange 222c and the inner tilt 214c of the reinforcing portion 213c are substantially parallel with each other, so that the preload is more evenly applied to the elastic member 23c between the slanted surface 223c and the inner tilt 214c. Moreover, the two surfaces are faced with each other, which causes little variation in deformation or little variation in the amount of preload among the elastic members 23 in respective attaching holes 12-14, thereby contributing to the accuracy.

In the embodiment described above, though the bush according to the present invention is used for the front cross-member, it is obvious that the bush may be used for a rear cross-member and the identical advantage is available then.

## Claims

1. A bush (2, 2a, 2b, 2c) which can be press-fitted into a top aperture and a bottom aperture of a bush-attaching hole (11, 12, 13, 14) penetrating a suspension cross-member (1) and fixable to a vehicular body (3) by a stud (42) and a nut (41) for mounting the suspension cross-member (1) to the bottom of the vehicular body (3), comprising,
an outer member (21a, 21b, 21c) including:
a) an outer main body (211a, 211b, 211c) in substantially cylindrical shape with an axial length shorter than a half of the axial length of the attaching hole (11, 12, 13, 14) of the suspension cross-member (1) and
b) an outer flange (212a, 212b, 212c) extending in the radially outward direction from an axial end of the outer main body (211a, 211b, 211 c),
an inner member (22a, 22b, 22c) including:
a) an inner main body (221a, 221b, 221c) in substantially cylindrical shape with an axial length longer than a half of the axial length of the attaching hole (11, 12, 13, 14) of the suspension cross-member (1) and with an external diameter smaller than the internal diameter of the outer main body (211a, 211b, 211c),
b) an inner flange (222a, 222b, 222c) extending in the radially outward direction from an axial end of the inner main body (221a, 221b, 221 c) with an external diameter larger than the internal diameter of the outer main body (211a, 211b, 211c), and
c) a through hole (224a, 224b, 224c) through which the stud (42) can be inserted, and the inner member (22a, 22b, 22c) being coaxially inserted into the outer member (21a, 21b, 21c) so that the inner flange (222a, 222b, 222c) and the outer flange (212a, 212b, 212c) are on the same side with respect to the axial direction, and
an elastic member (23a, 23b, 23c) at least partly disposed between the inner member (22a, 22b, 22c) and the outer member (21a, 21b, 21c) and adhered to the inner main body (221a, 221b, 221c), inner flange (222a, 222b, 222c), the outer main body (211a, 211b, 211 c), and the outer flange (212a, 212b, 212c),
**characterized in that** the outer member (21a, 21b, 21c), the inner member (22a, 22b, 22c) and/or the elastic member (23a, 23b, 23c) are formed such that
when the bushes (2, 2a, 2b, 2c) are press-fitted into the top aperture and the bottom aperture of the bush-attaching hole (11, 12, 13, 14) until the outer flanges (212a, 212b, 212c) contact with the suspension cross-member (1) before the bushes (2, 2a, 2b, 2c) are fixed by the stud (42) and the nut (41), the elastic members (23a, 23b, 23c) are substantially free from elastic deformation and provide a predetermined clearance (L) between the other axial ends of the inner members (22a, 22b, 22c), and
when the bushes (2, 2a, 2b, 2c) are fixed to the body (3) by the stud (42) and the nut (41), the elastic members (23a, 23b, 23c) are elastically deformed by tightening the nut (41) fitted onto the stud (42) so that the inner members (22a, 22b, 22c) are axially displaced with respect to the outer members (21a, 21b, 21c) to abut on each other.

2. A bush (2, 2a, 2b, 2c) as defined in claim 1, wherein said outer member (21a, 21 b, 21 c) further includes,
a jig-abutting portion (216a, 216b, 216c) provided in the outer flange (212a, 212b, 212c), on which a jig (5) can abut for press-fitting the bush (2, 2a, 2b, 2c) into an attaching hole (11, 12, 13, 14) in the suspension cross-member (1), and
a reinforcing portion (213a, 213b, 213c) formed as a bending portion of the outer flange (212a, 212b, 212c), for preventing deformation of said outer member (21a, 21b, 21c) when the bush (2, 2a, 2b, 2c) is press-fitted with the jig (5).

3. A bush (2, 2a, 2b, 2c) as defined in claim 2, wherein,
the jig-abutting portion (216a, 216b, 216c) is a substantially planar surface which continues to and is at substantially right angle with the outer main body (211a, 211b, 211c) in a cross section including the central axis of the bush (2, 2a, 2b, 2c).

4. A bush (2, 2a, 2b, 2c) defined in claim 2 or claim 3, wherein,
the reinforcing portion (213a, 213b, 213c) includes a slanted surface (214a, 214b, 214c) which is at an angle larger than the right angle with the outer main body (211a, 211b, 211c), and
the inner flange (222a, 222b, 222c) includes a tilt (223a, 223b, 223c) which confronts the slanted surface (214a, 214b, 214c) substantially in parallel.

5. A suspension cross-member (1), including:
a bush-attaching hole (11, 12, 13, 14) which penetrates the suspension cross-member (1) to form a top aperture and a bottom aperture on the top surface and the bottom surface of the suspension cross-member (1), respectively, and
a pair of bushes (2, 2a, 2b, 2c) as defined in one of the preceding claims, which are press-fitted into the top aperture and the bottom aperture, respectively.

6. A group of bushes (2a, 2b, 2c) as defined in one of the preceding claims 1 to 4, each of the bushes (2a, 2b, 2c) can be press-fitted into top apertures and bottom apertures of at least three bush-attaching holes (11, 12, 13, 14) penetrating the suspension cross-member (1) and fixed to a vehicular body (3) by at least three pairs of a stud (42) and a nut (41) for mounting the suspension cross-member (1) to the body (3), the number of the bushes (2a, 2b, 2c) consisting the group being equal to twice the number of the bush-attaching holes (11, 12, 13, 14), wherein,
the group of the bushes (2a, 2b, 2c) is composed of a first bush (2a), a second bush (2b), and third bushes (2c),
the first bush (2a) includes a through hole (224a) with an internal diameter approximately equal to an external diameter of the stud (42),
the second bush (2b) is substantially identical to the first bush (2a) excluding that the through hole (224b) of the second bush (2b) is oblong in cross-section which is expand in one radial direction from the through hole (224a) of the first bush (2a), and
the third bushes (2c) are substantially identical to the first bush (2a) excluding that the through hole (224c) of the third bushes (2c) are circular which is expanded in all radial directions from the through hole (224a) of the first bush (2a).

7. A suspension cross-member (1), including,
at least three bush-attaching holes (11, 12, 13, 14) which penetrate the suspension cross-member (1) to form top apertures and bottom apertures on the top surface and the bottom surface of the suspension cross-member (1), respectively, wherein, each bush (2a, 2b, 2c) of the group as defined in claim 6 is assigned and press-fitted in such a manner that:
the first bush (2a) is press-fitted into one of the top aperture and the bottom aperture of the first bush-attaching hole (11) of the at least three bush-attaching holes (11, 12, 13, 14),
the second bush (2b) is press-fitted into one of the top aperture and the bottom aperture of the second bush-attaching hole (12) of the at least three bush-attaching holes (11, 12, 13, 14) so that the expanded direction of the through hole (224b) is oriented substantially towards the location of the first bush-attaching hole (11),
the third bushes (2c) are press-fitted into the other aperture of the first bush-attaching hole (11), the other aperture of the second bush-attaching hole (12), and both the top apertures and the bottom apertures of the other bush-attaching holes (13, 14).

8. A vehicle on which the suspension cross-member (1) as defined in claim 5 or claim 7 is mounted, wherein,
the suspension cross-member (1) is fixed to the vehicular body (3) via the stud (42) and the nut (41) and the bushes (2, 2a, 2b, 2c) press-fitted into the bush-attaching holes (11, 12, 13, 14) so that the other axial ends of the inner members (22a, 22b, 22c) of the bushes (2, 2a, 2b, 2c) in the respective top apertures and the other axial ends the inner members (22a, 22b, 22c) of the respective bushes (2, 2a, 2b, 2c) in the bottom apertures abut on each other.

9. A method for mounting a bush (2, 2a, 2b, 2c) according to one of the preceding claims 1 to 4 into a top aperture and a bottom aperture of a bush-attaching hole (11, 12, 13, 14) penetrating a suspension cross-member (1) and fixable to a vehicular body (3) by a stud (42) and a nut (41) for mounting the suspension cross-member (1) to the bottom of the vehicular body (3), comprising the following steps:
press-fitting the bushes (2, 2a, 2b, 2c) into the top aperture and the bottom aperture of the bush-attaching hole (11, 12, 13, 14) until the outer flanges (212a, 212b, 212c) contact with the suspension cross-member (1) before the bushes (2, 2a, 2b, 2c) are fixed by the stud (42) and the nut (41), whereby the elastic members (23a, 23b, 23c) are substantially free from elastic deformation and provide a predetermined clearance (L) between the other axial ends of the inner members (22a, 22b, 22c), and
elastically deforming the elastic members (23a, 23b, 23c) by tightening the nut (41) fitted onto the stud (42) so that the inner members (22a, 22b, 22c) are axially displaced with respect to the outer members (21a, 21b, 21c) to abut on each other for fixing the bushes (2, 2a, 2b, 2c) to the body (3) by the stud (42) and the nut (41).

10. A method for mounting a group of bushes (2a, 2b, 2c) according to claim 6 to a suspension cross-member (1) having at least three bush-attaching holes (11, 12, 13, 14) which penetrate the suspension cross-member (1) to form top apertures and bottom apertures on the top surface and the bottom surface of the suspension cross-member (1), respectively, comprising the following steps:
press-fitting the first bush (2a) into one of the top aperture and the bottom aperture of the first bush-attaching hole (11) of the at least three bush-attaching holes (11, 12, 13, 14),
press-fitting the second bush (2b) into one of the top aperture and the bottom aperture of the second bush-attaching hole (12) of the at least three bush-attaching holes (11, 12, 13, 14) so that the expanded direction of the through hole (224b) is oriented substantially towards the location of the first bush-attaching hole (11), and
press-fitting the third bushes (2c) into the other aperture of the first bush-attaching hole (11), the other aperture of the second bush-attaching hole (12), and both the top apertures and the bottom apertures of the other bush-attaching holes (13, 14).

## Patentansprüche

1. Buchse bzw. Hülse (2, 2a, 2b, 2c), welche in eine obere bzw. Oberseitenöffnung und eine untere bzw. Bodenöffnung eines Buchsen-Festlegungslochs (11, 12, 13, 14) preßgepaßt werden kann, welches ein Querglied bzw. einen Querträger (1) einer Aufhängung durchdringt, und an einem Fahrzeugkörper bzw. einer Fahrzeugkarosserie (3) durch einen Bolzen bzw. Stift bzw. Nase bzw. Schaftschraube (42) und eine Mutter (41) zum Montieren des Querträgers (1) der Aufhängung an dem Boden der Fahrzeugkarosserie (3) fixierbar ist, umfassend,
ein äußeres bzw. Außenglied (21a, 21b, 21c) beinhaltend:
a) einen äußeren Hauptkörper (211a, 211b, 211c) in im wesentlichen zylindrischer Form mit einer axialen Länge kürzer als eine Hälfte der axialen Länge des Festlegungslochs (11, 12, 13, 14) des Querträgers (1) der Aufhängung und
b) einen äußeren Flansch (212a, 212b, 212c), welcher sich in der radial auswärts gerichteten Richtung von einem axialen Ende des äußeren Hauptkörpers (211a, 211b, 211c) erstreckt,
ein inneres bzw. Innenglied (22a, 22b, 22c) beinhaltend:
a) einen inneren Hauptkörper (221a, 221b, 221c) in einer im wesentlichen zylindrischen Form mit einer axialen Länge länger als eine Hälfte der axialen Länge des Festlegungslochs (11, 12, 13, 14) des Querträgers (1) der Aufhängung und mit einem Außendurchmesser geringer als der Innendurchmesser des äußeren Hauptkörpers (211a, 211b, 211c),
b) einen Innenflansch (222a, 222b, 222c), welcher sich in der radial auswärts gerichteten Richtung von einem axialen Ende des inneren Hauptkörpers (221a, 221b, 221c) mit einem Außendurchmesser größer als dem Innendurchmesser des äußeren Hauptkörpers (211a, 211b, 211c) erstreckt, und
c) ein Durchtrittsloch (224a, 224b, 224c), durch welches der Bolzen (42) eingesetzt werden kann, und wobei das innere Glied (22a, 22b, 22c) koaxial in das äußere Glied (21a, 21b, 21c) eingesetzt ist, so daß der innere Flansch (222a, 222b, 222c) und der äußere Flansch (212a, 212b, 212c) an derselben Seite in bezug auf die axiale Richtung liegen, und
ein elastisches Glied (23a, 23b, 23c), welches wenigstens teilweise zwischen dem inneren Glied (22a, 22b, 22c) und dem äußeren Glied (21a, 21b, 21c) angeordnet ist und an dem inneren Hauptkörper (221a, 221 b, 221c), dem inneren Flansch (222a, 222b, 222c), dem äußeren Hauptkörper (211a, 211b, 211c) und dem äußeren Flansch (212a, 212b, 212c) angehaftet bzw. festgelegt ist,
**dadurch gekennzeichnet, daß** das äußere Glied (21a, 21b, 21c), das innere Glied (22a, 22b, 22c) und/oder das elastische Glied (23a, 23b, 23c) derart ausgebildet sind, daß,
wenn die Buchsen (2, 2a, 2b, 2c) in die Oberseitenöffnung und die Bodenöffnung des Buchsen-Festlegungslochs (11, 12, 13, 14) preßgepaßt sind, bis die äußeren Flansche (212a, 212b, 212c) den Querträger (1) der Aufhängung kontaktieren, bevor die Buchsen (2, 2a, 2b, 2c) durch den Bolzen (42) und die Mutter (41) fixiert sind, die elastischen Glieder (23a, 23b, 23c) im wesentlichen frei von einer elastischen Deformation sind und einen vorbestimmten Abstand bzw. Freiraum (L) zwischen den anderen axialen Enden der inneren Glieder (22a, 22b, 22c) zur Verfügung stellen, und
wenn die Buchsen (2, 2a, 2b, 2c) an dem Körper (3) durch den Bolzen (42) und die Mutter (41) fixiert sind, die elastischen Glieder (23a, 23b, 23c) elastisch durch ein Anziehen der Mutter (41) deformiert sind bzw. werden, welche auf den Bolzen (42) gepaßt ist, so daß die inneren Glieder (22a, 22b, 22c) axial in bezug auf die äußeren Glieder (21a, 21b, 21c) verschoben bzw. verlagert sind, um aneinander anzuliegen bzw. anzuschlagen.

2. Buchse (2, 2a, 2b, 2c) nach Anspruch 1, wobei das äußere Glied (21a, 21b, 21c) weiters beinhaltet
einen Betätigungselement- bzw. Werkzeug-Anschlagabschnitt (216a, 216b, 216c), welcher in dem äußeren Flansch (212a, 212b, 212c) vorgesehen ist, an welchem ein Betätigungselement bzw. Werkzeug (5) für ein Preßpassen der Buchse (2, 2a, 2b, 2c) in ein Festlegungsloch (11, 12, 13, 14) in dem Querträger (1) der Aufhängung anliegen bzw. anschlagen kann, und
einen verstärkenden bzw. Verstärkungsabschnitt (213a, 213b, 213c), welcher als ein Biegeabschnitt des äußeren Flansches (212a, 212b, 212c) ausgebildet ist, um eine Deformation des äußeren Glieds (21a, 21b, 21c) zu verhindern, wenn die Buchse (2, 2a, 2b, 2c) mit dem Werkzeug (5) preßgepaßt ist.

3. Buchse (2, 2a, 2b, 2c) nach Anspruch 2, wobei
der Betätigungselement-Anschlagabschnitt (216a, 216b, 216c) eine im wesentlichen planare Fläche bzw. Oberfläche ist, welche an den äußeren Hauptkörper (211a, 211 b, 211 c) in einem Querschnitt anschließt und unter im wesentlichen rechten Winkel dazu ist, und zwar die zentrale Achse der Buchse (2, 2a, 2b, 2c) enthaltend.

4. Buchse (2, 2a, 2b, 2c) nach Anspruch 2 oder Anspruch 3, wobei
der verstärkende Abschnitt (213a, 213b, 213c) eine geneigte bzw. abgeschrägte Oberfläche (214a, 214b, 214c) beinhaltet, welche sich unter einem Winkel größer als der rechte Winkel mit dem äußeren Hauptkörper (211a, 211b, 211c) befindet, und
der innere Flansch (222a, 222b, 222c) eine Neigung bzw. Schrägstellung (223a, 223b, 223c) beinhaltet, welche der abgeschrägten Oberfläche (214a, 214b, 214c) im wesentlichen parallel gegenüberliegt.

5. Querträger bzw. -glied (1) einer Aufhängung, beinhaltend:
ein Buchsen-Festlegungsloch (11, 12, 13, 14), welches den Querträger (1) der Aufhängung durchdringt, um eine obere bzw. Oberseitenöffnung und eine untere bzw. Bodenöffnung an der oberen Fläche bzw. Oberfläche und der Bodenfläche bzw. -oberfläche des Querträgers (1) der Aufhängung jeweils auszubilden, und
ein Paar von Buchsen (2, 2a, 2b, 2c), wie sie in einem der vorangehenden Ansprüche definiert sind, welche in die Oberseitenöffnung und die Bodenöffnung jeweils preßgepaßt sind.

6. Gruppe von Buchsen (2a, 2b, 2c), wie sie in einem der vorangehenden Ansprüche 1 bis 4 definiert sind, wobei jede der Buchsen (2a, 2b, 2c) in obere bzw. Oberseitenöffnungen und untere bzw. Bodenöffnungen von wenigstens drei Buchsen-Festlegungslöchem (11, 12, 13, 14) preßgepaßt werden kann, welche das Querglied (1) der Aufhängung durchdringen und an einem Fahrzeugkörper bzw. einer Fahrzeugkarosserie (3) durch wenigstens drei Paare eines Bolzens bzw. Stift bzw. Nase bzw. Schaftschraube (42) und einer Mutter (41) zum Montieren des Querträgers (1) der Aufhängung an dem Körper (3) festgelegt sind, wobei die Anzahl der Buchsen (2a, 2b, 2c), welche die Gruppe bilden, das Doppelte einer Anzahl von Buchsen-Festlegungslöchem (11, 12, 13, 14) ist, wobei
die Gruppe der Buchsen (2a, 2b, 2c) aus einer ersten Buchse (2a), einer zweiten Buchse (2b) und dritten Buchsen (2c) gebildet bzw. zusammengesetzt ist,
die erste Buchse (2a) ein Durchtrittsloch (224a) mit einem Innendurchmesser ungefähr gleich einem Außendurchmesser des Bolzen (42) beinhaltet,
die zweite Buchse (2b) im wesentlichen identisch zu der ersten Buchse (2a) mit Ausnahme dahingehend ist, daß das Durchtrittsloch (224b) der zweiten Buchse (2b) im Querschnitt länglich ist, welcher sich in einer radialen Richtung von dem Durchtrittsloch (224a) der ersten Buchse (2a) erstreckt, und
die dritten Buchsen (2c) im wesentlichen identisch zu der ersten Buchse (2a) mit der Ausnahme sind, daß das Durchtrittsloch (224c) der dritten Buchsen (2c) kreisförmig ist, welches in alle radialen Richtungen von dem Durchtrittsloch (224a) der ersten Buchse (2a) aufgeweitet ist.

7. Querträger (1) einer Aufhängung, beinhaltend:
wenigstens drei Buchsen-Festlegungslöcher (11, 12, 13, 14), welche den Querträger (1) der Aufhängung durchdringen, um jeweils obere bzw. Oberseitenöffnungen und untere bzw. Bodenöffnungen an der oberen bzw. Oberseitenfläche bzw. -oberfläche und der unteren bzw. Bodenfläche bzw. -oberfläche des Querträgers (1) der Aufhängung auszubilden, wobei jede Buchse (2a, 2b, 2c) der Gruppe, wie sie in Anspruch 6 definiert ist, zugeteilt und in einer derartigen Weise preßgepaßt ist, das:
die erste Buchse (2a) in eine der oberen Öffnung und der Bodenöffnung des ersten Buchsen-Festlegungslochs (11) der wenigstens drei Buchsen-Festlegungslöcher (11, 12, 13, 14) preßgepaßt ist,
die zweite Buchse (2b) in eine der oberen Öffnung und der Bodenöffnung des zweiten Buchsen-Festlegungslochs (12) der wenigstens drei Buchsen-Festlegungslöcher (11, 12, 13, 14) preßgepaßt ist, so daß die aufgeweitete Richtung des Durchtrittslochs (224b) im wesentlichen in Richtung zu der Stelle des ersten Buchsen-Festlegungslochs (11) gerichtet bzw. orientiert ist,
die dritten Buchsen (2c) in die andere Öffnung des ersten Buchsen-Festlegungslochs (11), die andere Öffnung des zweiten Buchsen-Festlegungslochs (12) und sowohl die oberen Öffnungen als auch die Bodenöffnungen der anderen Buchsen-Festlegungslöcher (13, 14) preßgepaßt sind.

8. Fahrzeug, an welchem der Querträger (1) der Aufhängung, wie er in Anspruch 5 oder Anspruch 7 definiert ist, montiert ist, wobei
der Querträger (1) der Aufhängung an dem Fahrzeugkörper (3) über den Bolzen (42) und die Mutter (41) und die Buchsen (2, 2a, 2b, 2c) fixiert bzw. festgelegt ist, welche in die Buchsen-Festlegungslöcher (11, 12, 13, 14) preßgepaßt sind, so daß die anderen axialen Enden der inneren Glieder (22a, 22b, 22c) der Buchsen (2, 2a, 2b, 2c) in den entsprechenden oberen Öffnungen und die anderen axialen Enden der inneren Glieder (22a, 22b, 22c) der entsprechenden Buchsen (2, 2a, 2b, 2c) in den Bodenöffnungen aneinander anliegen bzw. anschlagen.

9. Verfahren zum Montieren einer Buchse (2, 2a, 2b, 2c) nach einem der vorangehenden Ansprüche 1 bis 4 in eine obere bzw. Oberseitenöffnung und eine untere bzw. Bodenöffnung eines Buchsen-Festlegungslochs (11, 12, 13, 14), welches einen Querträger (1) einer Aufhängung durchdringt und an einer Fahrzeugkarosserie bzw. einem Fahrzeugkörper (3) durch einen Bolzen (42) und eine Mutter (41) zum Montieren des Querträgers (1) der Aufhängung an dem Boden des Fahrzeugkörpers (3) festlegbar ist, umfassend die folgenden Schritte:
Preßpassen der Buchsen (2, 2a, 2b, 2c) in die obere Öffnung und die Bodenöffnung des Buchsen-Festlegungslochs (11, 12, 13, 14), bis die äußeren Flansche (212a, 212b, 212c) den Querträger (1) der Aufhängung kontaktieren, bevor die Buchsen (2, 2a, 2b, 2c) durch den Bolzen (42) und die Mutter (41) fixiert sind, wodurch die elastischen Glieder (23a, 23b, 23c) im wesentlichen frei von einer elastischen Deformation sind und einen vorbestimmten Abstand bzw. Freiraum (L) zwischen den anderen axialen Enden der inneren Glieder (22a, 22b, 22c) zur Verfügung stellen, und elastisches Deformieren bzw. Verformen der elastischen Glieder (23a, 23b, 23c) durch ein Festziehen der Mutter (41), welche auf dem Bolzen (42) gepaßt wird, so daß die inneren Glieder (22a, 22b, 22c) axial in bezug auf die äußeren Glieder (21a, 21b, 21c) verschoben bzw. verlagert werden, um aneinander für ein Fixieren bzw. Festlegen der Buchsen (2, 2a, 2b, 2c) an dem Körper (3) durch den Bolzen (42) und die Mutter (41) anzuliegen.

10. Verfahren zum Montieren einer Gruppe von Buchsen (2a, 2b, 2c) gemäß Anspruch 6 an einem Querträger (1) einer Aufhängung, welcher wenigstens drei Buchsen-Festlegungslöcher (11, 12, 13, 14) aufweist, welche den Querträger (1) der Aufhängung durchdringen, um jeweils obere bzw. Oberseitenöffnungen und untere bzw. Bodenöffnungen an der oberen Fläche bzw. Oberfläche und der Bodenfläche bzw. -oberfläche des Querträgers (1) der Aufhängung auszubilden, umfassend die folgenden Schritte:
Preßpassen der ersten Buchse (2a) in eine der oberen Öffnung und der Bodenöffnung des ersten Buchsen-Festlegungslochs (11) der wenigstens drei Buchsen-Festlegungslöcher (11, 12, 13, 14),
Preßpassen der zweiten Buchse (2b) in eine der oberen Öffnung und der Bodenöffnung des zweiten Buchsen-Festlegungsloch (12) der wenigstens drei Buchsen-Festlegungslöcher (11, 12, 13, 14), so daß die aufgeweitete Richtung des Durchtrittslochs (224b) im wesentlichen in Richtung zu der Stelle des ersten Buchsen-Festlegungslochs (11) orientiert ist, und
Preßpassen der dritten Buchsen (2c) in die andere Öffnung des ersten Buchsen-Festlegungslochs (11), die andere Öffnung des zweiten Buchsen-Festlegungslochs (12), und sowohl die oberen Öffnungen als auch die Bodenöffnungen der anderen Buchsen-Festlegungslöcher (13, 14).

## Revendications

1. Manchon (2, 2a, 2b, 2c) qui peut être ajusté sous pression dans une ouverture supérieure et dans une ouverture inférieure d'un trou de fixation (11, 12, 13, 14) de manchon qui pénètre dans un élément transversal de suspension (1) et qui peut être fixé sur un corps (3) de véhicule par un goujon (42) et un écrou (41) de manière à monter l'élément transversal de suspension (1) sur la partie inférieure du corps (3) de véhicule, qui comprend :
un élément extérieur (21a, 21b, 21c) qui comprend :
a) un corps extérieur principal (211a, 211b, 211c) qui a une forme essentiellement cylindrique et dont la longueur axiale est plus courte que la moitié de la longueur axiale du trou de fixation (11, 12, 13, 14) de l'élément transversal de suspension (1) et
b) une bride extérieure (212a, 212b, 212c) qui s'étend dans la direction qui va radialement vers l'extérieur par rapport à l'extrémité axiale du corps extérieur principal (211a, 211b, 211c),
un élément intérieur (22a, 22b, 22c) qui comprend :
a) un corps intérieur principal (221a, 221b, 221c) qui a une forme essentiellement cylindrique, dont la longueur axiale est plus longue que la moitié de la longueur axiale du trou de fixation (11, 12, 13, 14) de l'élément transversal de suspension (1) et dont le diamètre extérieur est plus petit que le diamètre intérieur du corps extérieur principal (211a, 211b, 211c),
b) une bride intérieure (222a, 222b, 222c) qui s'étend dans la direction qui va radialement vers l'extérieur par rapport à l'extrémité axiale du corps intérieur principal (221a, 221b, 221c) et dont le diamètre extérieur est plus grand que le diamètre intérieur du corps extérieur principal (211a, 211b, 211c) et
c) une perforation (224a, 224b, 224c) dans laquelle le goujon (42) peut être logé, l'élément intérieur (22a, 22b, 22c) étant logé coaxialement dans l'élément extérieur (21a, 21b, 21c) de telle sorte que la bride intérieure (222a, 222b, 222c) et la bride extérieure (212a, 212b, 212c) soient du même côté par rapport à la direction axiale, et
un élément souple (23a, 23b, 23c) qui est disposé au moins en partie entre l'élément intérieur (22a, 22b, 22c) et l'élément extérieur (21a, 21b, 21c) et qui est collé sur le corps intérieur principal (221a, 221 b, 221c), la bride intérieure (222a, 222b, 222c), le corps extérieur principal (211a, 211b, 211 c) et la bride extérieure (212a, 212b, 212c),
**caractérisé en ce que** l'élément extérieur (21a, 21b, 21c), l'élément intérieur (22a, 22b, 22c) et/ou l'élément souple (23a, 23b, 23c) sont formés de telle sorte que :
lorsque les manchons (2, 2a, 2b, 2c) sont insérés sous pression dans l'ouverture supérieure et dans l'ouverture inférieure du trou de fixation (11, 12, 13, 14) de manchon jusqu'à ce que les brides extérieures (212a, 212b, 212c) soient en contact avec l'élément transversal de suspension (1) avant que les manchons (2, 2a, 2b, 2c) aient été fixés par le goujon (42) et l'écrou (41), les éléments souples (23a, 23b, 23c) sont essentiellement dépourvus de déformation élastique et constituent un interstice prédéterminé (L) entre les autres extrémités axiales des éléments intérieurs (22a, 22b, 22c), et que
lorsque les manchons (2, 2a, 2b, 2c) sont fixés sur le corps (3) par le goujon (42) et l'écrou (41), les éléments souples (23a, 23b, 23c) sont élastiquement déformés en serrant l'écrou (41) inséré sur le goujon (42) de telle sorte que les éléments intérieurs (22a, 22b, 22c) se déplacent axialement par rapport aux éléments extérieurs (21a, 21 b, 21 c) de manière à buter les uns contre les autres.

2. Manchon (2, 2a, 2b, 2c) selon la revendication 1, dans lequel ledit élément extérieur (21a, 21b, 21c) comprend en outre :
une partie (216a, 216b, 216c) de butée de monture prévue dans la bride extérieure (212a, 212b, 212c), contre laquelle une monture (5) peut buter de manière à insérer sous pression le manchon (2, 2a, 2b, 2c) dans un trou de fixation (11, 12, 13, 14) ménagé dans l'élément transversal de suspension (1) et
une partie de renforcement (213a, 213b, 213c) sous la forme d'une partie pliée de la bride extérieure (212a, 212b, 212c) qui empêche la déformation dudit élément extérieur (21a, 21b, 21c) lorsque le manchon (2, 2a, 2b, 2c) est inséré sous pression dans la monture (5).

3. Manchon (2, 2a, 2b, 2c) selon la revendication 2, dans lequel la partie (216a, 216b, 216c) de butée de monture est une surface essentiellement plane qui continue jusqu'au corps extérieur principal (211a, 211 b, 211 c) et qui est essentiellement perpendiculaire à ce dernier dans une section transversale qui comprend l'axe central du manchon (2, 2a, 2b, 2c).

4. Manchon (2, 2a, 2b, 2c) selon les revendications 2 ou 3, dans lequel la partie de renforcement (213a, 213b, 213c) comprend une surface inclinée (214a, 214b, 214c) qui est inclinée sous un angle supérieur à l'angle droit formé avec le corps extérieur principal (211a, 211b, 211c), la bride intérieure (222a, 222b, 222c) comprenant une inclinaison (223a, 223b, 223c) qui fait face à la surface inclinée (214a, 214b, 214c) et lui est essentiellement parallèle.

5. Elément transversal de suspension (1), qui comprend :
un trou de fixation (11, 12, 13, 14) de manchon qui pénètre dans l'élément transversal de suspension (1) de manière à former une ouverture supérieure et une ouverture inférieure respectivement sur la surface supérieure et sur la surface inférieure de l'élément transversal de suspension (1) et
deux manchons (2, 2a, 2b, 2c) selon l'une des revendications précédentes qui sont insérés sous pression respectivement dans l'ouverture supérieure et l'ouverture inférieure.

6. Ensemble de manchons (2a, 2b, 2c) selon l'une des revendications précédentes 1 à 4, les manchons (2a, 2b, 2c) pouvant chacun être insérés sous pression dans les ouvertures supérieures et dans les ouvertures inférieures d'au moins trois trous de fixation (11, 12, 13, 14) de manchon qui pénètrent dans l'élément transversal de suspension (1) et qui sont fixés sur un corps (3) de véhicule par au moins trois paires constituées d'un goujon (42) et d'un écrou (41) pour monter l'élément transversal de suspension (1) sur le corps (3), le nombre dé manchons (2a, 2b, 2c) qui constitue l'ensemble étant égal à deux fois le nombre de trous de fixation (11, 12, 13, 14) de manchon, dans lequel l'ensemble de manchons (2a, 2b, 2c) est constitué d'un premier manchon (2a), d'un deuxième manchon (2b) et de troisièmes manchons (2c), lé premier manchon (2a) comprenant une perforation (224a) dont le diamètre intérieur est environ égal au diamètre extérieur du goujon (42), le deuxième manchon (2b) étant essentiellement identique au premier manchon (2a), à la différence que la perforation (224a) du deuxième manchon (2b) présente une section transversale oblongue et qu'elle est élargie dans la direction radiale par rapport à la perforation (224a) du premier manchon (2a), et les troisièmes manchons (2c) étant essentiellement identiques au premier manchon (2a), à la différence que la perforation (224a) des troisièmes manchons (2c) est circulaire et qu'elle est élargie dans toutes les directions radiales par rapport à la perforation (224a) du premier manchon (2a).

7. Elément transversal de suspension (1), qui comprend :
au moins trois trous de fixation (11, 12, 13, 14) de manchon qui pénètrent.dans l'élément transversal de suspension (1) de manière à former des ouvertures supérieures et des ouvertures inférieures respectivement sur la surface supérieure et sur la surface inférieure de l'élément transversal de suspension (1), chaque manchon (2a, 2b, 2c) de l'ensemble selon la revendication 6 étant attribué et inséré sous pression de telle sorte que :
le premier manchon (2a) est inséré sous pression dans l'une parmi l'ouverture supérieure et l'ouverture inférieure du premier trou de fixation (12) de manchon des trois trous de fixation (11, 12, 13, 14) de manchon au moins présents,
le deuxième manchon (2b) est inséré sous pression dans l'une parmi ouverture supérieure et l'ouverture inférieure du deuxième trou de fixation (12) de manchon des trois trous de fixation (11, 12, 13, 14) au moins présents de telle sorte que la direction d'élargissement de la perforation (224b) est orientée essentiellement vers l'emplacement du premier trou de fixation (11) de manchon,
les trois manchons (2c) sont insérés sous pression dans l'autre ouverture du premier trou de fixation (11) de manchon, dans l'autre ouverture du deuxième trou de fixation (12) de manchon et dans les ouvertures supérieures et les ouvertures inférieures des autres trous de fixation (13, 14) de manchon.

8. Véhicule sur lequel est monté l'élément transversal de suspension (1) selon les revendications 5 ou 7, dans lequel l'élément transversal de suspension (1) est fixé sur le corps (3) de véhicule par l'intermédiaire du goujon (42) et de l'écrou (41), les manchons (2, 2a, 2b, 2c) étant insérés sous pression dans les trous de fixation (11, 12, 13, 14) de telle sorte que les autres extrémités axiales des éléments intérieurs (22a, 22b, 22c) des manchons (2, 2a, 2b, 2c) situées dans les ouvertures supérieurs respectives et les autres extrémités axiales des éléments intérieurs (22a, 22b, 22c) des manchons respectifs (2, 2a, 2b, 2c) situées dans les ouvertures inférieures butent les unes contre les autres.

9. Procédé pour monter un manchon (2, 2a, 2b, 2c) selon l'une des revendications précédentes 1 à 4 dans une ouverture supérieure et une ouverture inférieure d'un trou de fixation (11, 12, 13, 14) de manchon qui pénètrent dans un élément transversal de suspension (1) qui peut être fixé sur un corps (3) de véhicule par un goujon (42) et un écrou (41) de manière à monter l'élément transversal de suspension (1) sur la partie inférieure du corps (3) du véhicule, qui comprend les étapes qui consistent à :
insérer sous pression les manchons (2, 2a, 2b, 2c) dans l'ouverture supérieure et l'ouverture inférieure du trou de fixation (11, 12, 13, 14) de manchon jusqu'à ce que les brides extérieures (212a, 212b, 212c) soient en contact avec l'élément transversal de suspension (1) avant que les manchons (2, 2a, 2b, 2c) ont été fixés par le goujon (42) et l'écrou (41), les éléments souples (23a, 23b, 23c) étant essentiellement dépourvus de déformation élastique et constituant un interstice prédéterminé (L) entre les autres extrémités axiales des éléments intérieurs (22a, 22b, 22c) et
déformer élastiquement les éléments souples (23a, 23b, 23c) en serrant l'écrou (41) inséré sur le goujon (42) de telle sorte que les éléments intérieurs (22a, 22b, 22c) se déplacent axialement par rapport aux éléments extérieurs (21a, 21b, 21c) de manière à buter les uns contre les autres en vue de fixer les manchons (2, 2a, 2b, 2c) sur le corps (3) à l'aide du goujon (42) et de l'écrou (41).

10. Procédé pour monter un ensemble de manchons (2a, 2b, 2c) selon la revendication 6 sur un élément transversal de suspension (1) qui présente au moins trois trous de fixation (11, 12, 13, 14) de manchon qui pénètrent dans l'élément transversal de suspension (1) de manière à former des ouvertures supérieures et des ouvertures inférieures respectivement sur la surface supérieure et la surface inférieure de l'élément transversal de suspension (1), qui comprend les étapes qui consistent à :
insérer sous pression le premier manchon (2a) dans l'une parmi l'ouverture supérieure et l'ouverture inférieure du premier trou de fixation (11) de manchon des trois trous de fixation (11, 12, 13, 14) de manchon au moins présents,
insérer sous pression le deuxième manchon (2b) dans l'une parmi l'ouverture supérieure et l'ouverture inférieure du deuxième trou de fixation (12) de manchon des trois trous de fixation (11, 12, 13, 14) de manchon au moins présents de telle sorte que la direction d'élargissement de la perforation (224b) soit orientée essentiellement vers l'emplacement du premier trou de fixation (11) de manchon et
insérer sous pression les troisièmes manchons (2c) dans l'autre ouverture du premier trou de fixation (11) de manchon, dans l'autre ouverture du deuxième trou de fixation (12) de manchon et dans les ouvertures supérieures et les ouvertures inférieures des autres trous de fixation (13, 14) de manchon.
